# EUROPEAN PATENT SPECIFICATION

(11) **EP 0 442 026 B1**
(45) Date of publication and mention of the grant of the patent: **27.12.1995**
(21) Application number: 90111949.5
(22) Date of filing: 23.06.1990
(51) Int. Cl.: C07H 1/08, C12N 15/10

(54) **Method to extract and purify human genomic DNA**
Verfahren zur Trennung und Reinigung menschlicher, genomischer DNS
Procédé pour extraire et purifier de l'ADN génomique humain

(30) Priority: 14.02.1990 IT 8333490
(43) Date of publication of application: 21.08.1991
(73) Proprietor: Talent SRL, I-34148 Trieste (IT)
(72) Inventor: Schneider, Claudio, I-33100 Udine (IT)
(74) Representative: Alagem Modiano, Lara S.

(56) References cited:
- US-A- 3 176 006
- RESEARCH IN MOLECULAR BIOLOGY, vol. 3, 1974, pages 93-166, Akademie der Wissenschaften und der Literatur, Mainz, DE; W. HÖNIG et al.: "Desoxyribonucleinsäure-Isolierung"

## Description

This invention concerns a method to extract and purify genomic DNA from human total blood, tissues, sperm, cultured cells. The same method can be used also for extracting DNA from animal blood and tissues, virus (es. hepatitis), vegetal cells.

To be more exact, the invention provides for a method which can be partly reproduced manually and partly automatically in limited performance times.

The traditional method employed, for instance, by the "DNA Extractor" apparatus of the Applied Biosystem is known but is not satisfactory owing to its low percentage yield and its slowness; moreover, it employs as a reagent phenol which is expansive, irritant and neurotoxic. The present applicant has therefore tackled the problem of providing a method which enables higher percentage yields to be achieved more speedly and simply.

Known from Honig et al, Research in Molecular Biology, Volume 3, 1974, pages 93-166, are other effective methods for extracting and purifying genomic DNA, using, for example, the cationic detergent cetyltrimethylammonium bromide.

The method to extract and purify human genomic DNA is set forth and characterized in the main claim, while the dependent claims describe variants of the idea of the main solution.

The method according to the invention makes it possible to obtain in about twenty minutes what can be obtained otherwise in about four hours with a lower yield.

Moreover the method according to the invention can be automated at least partly with a suitable apparatus which provides the human genomic DNA in 10 to 15 minutes in a test-tube containing a small quantity of water (500-1000 microlitres for instance).

The method, according to the invention, for the extraction and purification of DNA, starting from human blood as a whole, provides for at least the following steps:
- charging the sample, which undergoes a lysis treatment
- extracting the protein with an organic solvent, chloroform for instance
- centrifuging to eliminate the protein portion
- diluting in water and precipiting with cationic detergent
- filtering and adherence of the genomic DNA to a suitable filter
- washing the filter and eluting the genomic DNA, which is resuspended in water.

The DNA obtained in this way can be employed in futher analysis:
1. Enzimatic restriction-Southern blot.
2. Amplification of specific genic fragments with the polymerase chain reaction (PCR).

Percentage yields as compared to the traditional methods vary between 120% and 150%, with a recovery of 40 micrograms per millilitre of blood.

To be more exact, the method according to the invention includes the following main steps:
STEP 1: charging a sample of blood, normally between 300 and 2500 microlitres but also a different amount, which undergoes a treatment of lysis by a cationic detergent for example tetradecyltrimethilammoniumbromide (TTAB) or dodecyltrimethilammoniumbromide (DTAB) both with sodium cloride at a concentration higher than 0.5 M.
   This solution is mixed and heated up to 68°C and incubated for a while (es. five minutes).
STEP 2: an extraction is made by adding 1 volume of chloroform or other organic solvents.
STEP 3: the mixture undergoes centrifuging with a normal bench centrifuge for some minutes to eliminate the protein portion which forms a clog with the organic phase.
STEP 4: after centrifuging, to the acqueos phase are added a quantity of water to decrease the ionic strenght below 0,5 M NaCl, and a cationic detergent (for instance a solution of 5% of Cetyl-trimetil-ammonium bromide), so that precipitation of the cationic detergent micellar complex-DNA takes place after a short mixing operation.
STEP 5: the solution thus obtained and containing the micellar-DNA complex then undergoes filtration. The ultrafiltration takes place with a filter (for instance, sintered borosilicate glass or an organic matrix like polypropilene or polyethylene) of known and tested porosity (pore size between 5 and 15 micron), which retains the DNA-cationic detergent micellar complex in a satisfactory way. The hydrophilic surface enables DNA to be recovered easily and speedly after the washing operations. The organic matrix filter allows a slower recovery of DNA so it is not commonly used at the moment.
   As a genomic DNA is immobilized on the filter, it is then eluted.
STEP 6: this elution takes place after a series of washing of the filter:
   - a first wash is carried out in an acqueos solution of a low ionic strenght to eliminate the excess of detergent;
   - a second wash is carried out with a mixtures of alcohol and an ionic solution, for instance of the type NaCl 0,2 M/70% ethanol, to change the DNA-cationic detergent complex into DNA-Na;
   - a third wash is carried out with a mixture of alcohol and a solution of low ionic strenght to remove the excess of salts, for instance NaCl type (for instance, 70% ethanol and 30% water).
   After having removed every trace of alcohol from the filter by a current of air, the DNA is recovered by washing the filter with an acqueos solution of a low ionic strenght (water, for instance) at room temperature or by heating, for instance at 68°C.

## Claims

1. Method for extraction of genomic DNA from a material selected from whole blood, tissues, sperm, cultured cells, virus and vegetal cells, said method comprising the steps of:
1- subjecting a sample of said material to a lysis treatment by a first cationic detergent, selected from tetradecyltrimethylammoniumbromide and dodecyltrimethylamoniumbromide, with sodium chloride at a concentration higher than 0.5 M, followed by heating up to 68°C and incubation for about five minutes;
2- adding to the solution obtained in step 1 an organic solvent to extract the proteins contained in said sample;
3- centrifuging the mixture obtained in step 2 in a bench centrifuge to separate an organic phase containing proteins and an aqueous phase;
4- diluting said aqueous phase with water up to an ionic strength below 0.5 M of sodium chloride, and adding a second cationic detergent, thereby a cationic detergent micellar-DNA complex is formed;
5- separating said cationic detergent micellar-DNA complex by filtration with a filter with pore size from 5 to 15 micrometers;
6- washing said filter by carrying out a first wash with an aqueous solution of a low ionic strength to eliminate the excess of detergent; a second wash with a mixture of alcohol and a solution of sodium chloride, to change said cationic detergent micellar-DNA complex into DNA-Na; a third wash with a mixture of alcohol and a solution of low ionic strength to remove the excess of salts, and then removing of alcohol traces from said filter by a current of air and re-suspending of the DNA by washing the filter with an aqueous solution of low ionic strength at room temperature or by heating up to 68°C.

2. Method according to claim 1, characterized in that said material is whole blood.

3. Method according to claim 1, characterized in that said organic solvent added in step 2 is chloroform.

4. Method according to claim 1, characterized in that said second cationic detergent added in step 4 is a solution of cetyl-trimethyl-ammonium bromide.

5. Method according to claim 4, characterized in that said solution of cetyl-trimethyl-ammonium bromide has a concentration of 5%.

6. Method according to claim 1, characterized in that said filter is made of sintered borosilicate glass.

7. Method according to claim 1 characterized in that said filter is made of an organic matrix selected from polypropylene and polyethylene.

8. Method according to claim 1, characterized in that said second wash in step 6 is carried out with a mixture of sodium chloride 0.2 M/70% ethanol.

9. Method according to claim 1, characterized in that said third wash in step 6 is carried out with a mixture of 70% ethanol and 30% water.

10. Method according to claim 1, characterized in that said re-suspension of the DNA of step 6 is carried out by washing said filter with water.

## Patentansprüche

1. Verfahren zur Extraktion genomischer DNS aus einem Material, das aus vollständigem Blut, Geweben, Sperma, kultivierten Zellen, Viren und vegetalen Zellen ausgewählt ist, wobei das Verfahren folgende Schritte aufweist:
1- eine Probe des besagten Materials wird einer Lysis-Behandlung durch ein erstes kationisches Detergens unterworfen, das aus Tetradecyltrimethylammoniumbromid und Dodecyltrimethylammoniumbromid ausgewählt ist, mit Natriumchlorid bei einer Konzentration größer als 0,5 M, gefolgt von Aufheizen auf 68 °C und Inkubation für ungefähr fünf Minuten;
2- Zugabe eines organischen Lösungsmittels zur in Schritt 1 erhaltenen Lösung, um die in der Probe enthaltenen Proteine zu extrahieren;
3- Zentrifugieren der in Schritt 2 erhaltenen Mischung in einer Laborzentrifuge, um eine Proteine enthaltende organische Phase und ein wässrige Phase zu trennen;
4- Verdünnen der wässrigen Phase mit Wasser bis zu einer Ionenstärke kleiner 0,5 M Natriumchlorid und Zugabe eines zweiten kationischen Detergens, wodurch ein kationischer Detergens-Mizellar-DNS-Komplex gebildet wird;
5- Separieren des kationischen Detergens-Mizellar-DNS-Komplexes durch Filtration mit einem Filter mit einer Porengröße von 5 »m bis 15 »m;
6- Waschen des Filters durch eine erste Waschung mit einer wässrigen Lösung einer geringen Ionenstärke, um den Detergens-Überschuß zu beseitigen; eine zweite Waschung mit einer Mischung aus Alkohol und einer Natriumchlorid-Lösung, um den kationischen Detergens-Mizellar-DNS-Komplex in DNS-Na umzuwandeln; eine dritte Waschung mit einer Mischung aus Alkohol und einer Lösung einer geringen Ionenstärke, um den Überschuß an Salzen zu entfernen, und anschließendes Entfernen von Alkoholspuren aus dem Filter mittels eines Luftstroms und Resuspendieren der DNS durch Waschen des Filters mit einer wässrigen Lösung einer geringen Ionenstärke bei Raumtemperatur oder durch Aufheizen bis auf 68°C.

2. Verfahren nach Anspruch 1, **dadurch gekennzeichnet, daß** das Material vollständiges Blut ist.

3. Verfahren nach Anspruch 1, **dadurch gekennzeichnet, daß** das in Schritt 2 dazugegebene organische Lösungsmittel Chloroform ist.

4. Verfahren nach Anspruch 1, **dadurch gekennzeichnet, daß** das in Schritt 4 dazugegebene zweite kationische Detergens eine Lösung von Cetyl-Trimethyl-Ammoniumbromid ist.

5. Verfahren nach Anspruch 4, **dadurch gekennzeichnet, daß** die Cetyl-Trimethyl-Ammoniumbromid-Lösung eine Konzentration von 5% aufweist.

6. Verfahren nach Anspruch 1, **dadurch gekennzeichnet, daß** der Filter aus gesintertem Borosilikat-Glas hergestellt ist.

7. Verfahren nach Anspruch 1, **dadurch gekennzeichnet, daß** der Filter aus einer aus Polypropylen und Polyethylen ausgewählten organischen Matrix hergestellt ist.

8. Verfahren nach Anspruch 1, **dadurch gekennzeichnet, daß** die zweite Waschung in Schritt 6 mit einer Mischung aus 0,2 M Natriumchlorid/70% Ethanol durchgeführt wird.

9. Verfahren nach Anspruch 1, **dadurch gekennzeichnet, daß** die dritte Waschung in Schritt 6 mit einer Mischung aus 70% Ethanol und 30% Wasser durchgeführt wird.

10. Verfahren nach Anspruch 1, **dadurch gekennzeichnet, daß** das Resuspendieren der DNS in Schritt 6 durch Waschen des Filters mit Wasser durchgeführt wird.

## Revendications

1. Procédé pour l'extraction de DNA génomique, à partir d'un matériau choisit parmi du sang entier, des tissus, du sperme, des cellules sous culture, des virus et des cellules végétales, ledit procédé comprenant les étapes de :
1) Soumette un échantillon dudit matériau à un traitement de dissolution à l'aide d'un premier détergent cationique, choisit parmi le bromure de tétradecyltrimethylammonium et le bromure de dodecyltrimethylamonium, avec une concentration de chlorure de sodium supérieure à 0,5 M, suivi par une élévation en température jusqu'à 68°C, et une incubation pendant environ 5 mn;
2) Ajouter à la solution obtenue à l'étape 1, un solvant organique afin d'extraire les protéines contenues dans ledit échantillon;
3) Soumettre à centrifugation le mélange obtenu à l'étape n°2 dans une centrifuge à banc afin de séparer en une phase organique contenant des protéines et une phase aqueuse;
4) Diluer ladite phase aqueuse avec de l'eau jusqu'à une concentration en ion inférieure à 0,5 M de chlorure de sodium, et ajouter un second détergent cationique de manière à former un complexe DNA micellaire détergent cationique;
5) Séparer ledit complexe DNA micellaire détergent cationique par filtration avec un filtre présentant des tailles de pores de 5 à 15 microns;
6) Laver ledit filtre en effectuant un premier lavage à l'aide d'une solution aqueuse à faible concentration ionique afin d'éliminer l'excès de détergent; un second lavage avec un mélange d'alcool et de solution de chlorure de sodium, pour changer ledit complexe DNA micellaire détergent cationique en un DNA-sodium; un troisième levage avec une mélange d'alcool et une solution à faible concentration ionique, afin d'enlever l'excès de sel, et ensuite enlever les tracer d'alcool dudit filtre par un courant d'air et remise en suspension du DNA en lavant le filtre avec une solution aqueuse de faible concentration ionique à température ambiante ou par élévation de température jusqu'à 68° C.

2. Procédé selon la revendication 1, caractérisé en ce que ledit matériau est constitué de sang entier.

3. Procédé selon la revendication 1, caractérisé en ce que ledit solvant organique ajouté à l'étape 2 est constitué de chloroforme.

4. Procédé selon la revendication 1, caractérisé en ce que ledit second détergent cationique ajouté à l'étape 4 est une solution de bromure de cetyl-trimethylammonium.

5. Procédé selon la revendication 4, caractérisé en ce que ladite solution de bromure de cetyl-triméthylammonium présente une concentration de 5%.

6. Procédé selon la revendication 1, caractérisé en ce que ledit filtre est réalisé en verre de silicate de bore aggloméré.

7. Procédé selon la revendication 1, caractérisé en ce que ledit filtre est constitué d'une matrice organique choisie parmi le polypropylène et le polyéthylène.

8. Procédé selon la revendication 1, caractérisé en ce que ledit second lavage de l'étape 6 est réalisé à l'aide d'un mélange de chlorure de sodium à 0,2 M et 70% d'éthanol.

9. Procédé selon la revendication 1, caractérisé en ce que ledit troisième lavage de l'étape 6 est réalisé avec un mélange de 70% d'éthanol et 30% d'eau.

10. Procédé selon la revendication 1, caractérisé en ce que ladite remise en suspension du DNA de l'étape 6 est réalisée en lavant ledit filtre avec de l'eau.
